# EUROPEAN PATENT SPECIFICATION

(11) **EP 2 280 931 B1**
(45) Date of publication and mention of the grant of the patent: **11.07.2012**
(21) Application number: 09742061.6
(22) Date of filing: 05.05.2009
(51) Int. Cl.: C07C 227/28, C07C 229/40

(54) **PROCESS FOR CONVERTING TRYPTOPHAN INTO KYNURENINE**
VERFAHREN ZUR UMWANDLUNG VON TRYPTOPHAN IN KYNURENIN
PROCÉDÉ DE CONVERSION DU TRYPTOPHANE EN KYNURÉNINE

(30) Priority: 07.05.2008 US 51034 P
(43) Date of publication of application: 09.02.2011
(73) Proprietor: Huntsman International LLC, Salt Lake City, UT 84108 (US)
(72) Inventor: SANDERS, Johan, NL-9722 WL Groningen (NL); PETER, Francisc, R-300740 Timisoara (RO); SCOTT, Elinor, NL-3822 VK Amersfoort (NL); SAAD, Mohamed Hamdy, NL-2625 TP Delft (NL); CARR, Robert Henry, B-3060 Bertem (BE)
(74) Representative: Van Steenlandt, Wim August Maria
(86) International application number: PCT/EP2009/055419
(87) International publication number: WO 2009/135843

(56) References cited:
- ZHURNAL ORGANICHESKOI KHIMII, MAIK NAUKA, MOSCOW, RU, vol. 25, no. 2, 1 January 1989 (1989-01-01), pages 431-433, XP009122916 ISSN: 0514-7492
- MASAKO NAKAGAWA ET AL: "DYE-SENSITIZED PHOTO-OXYGENATION OF TRYPTOPHAN" TETRAHEDRON, vol. 41, no. 11, 1985, pages 2125-2132, XP002548899
- MASAKO NAKAGAWA ET AL: "3a-HYDROPEROXYPYRROLOINDOLE FROM TRYPTOPHAN. ISOLATION AND TRANSFORMATION TO FORMYLKYNURENINE." JOURNAL OF THE AMERICAN SOCIETY, vol. 101, no. 11, 1979, pages 3136-3137, XP002548900
- MASAKO NAKAGAWA ET AL: "A VALID MODEL FOR THE MECHANISM OF OXIDATION OF TRYPTOPHAN TO FORMYLKYNURENINE" PROC. NATL. ACAD. SCI. USA, vol. 74, no. 11, 1977, pages 4730-4733, XP002548901
- MASAKO NAKAGAWA; YUKIO YOKOYAMA; SHIRO KATA; AND TAHRU HINO: "Oxidative transformation of tryptophan to 5-hydroxy-n-formylkynurenine" HETEROCYCLES, ELSEVIER SCIENCE PUBLISHERS B.V. AMSTERDAM, NL, vol. 22, 1 January 1984 (1984-01-01), pages 59-62, XP009123475 ISSN: 0385-5414
- WILLARD G. MCCULLOUGH ET AL: "ENZYMATIC DECARBOXYLATION OF THE AMINOBENZOATES" JOURNAL OF THE AMERICAN SOCIETY, vol. 79, 1957, pages 628-630, XP002548902
- L.MCMASTER AND R.L.SHRINER: "THE DECOMPOSITION OF THE AMINOBENZOIC ACIDS BY BOILING WATER" JOURNAL OF THE AMERICAN SOCIETY, vol. 45, 1923, pages 751-753, XP002548903

## Description

The present invention relates to a process for the conversion of valuable rest streams or waste or primary agricultural streams or mixtures thereof which comprise proteins and/or amino acids into valuable chemicals. In particular, the present invention relates to a process for converting tryptophan into kynurenine. This converting process in its turn is suitable as a process step in a process for the manufacture of aniline from valuable rest streams or waste or primary agricultural streams or mixtures thereof, in particular from streams which comprise the amino acid tryptophan.

Many base chemicals are manufactured by the chemical industry on an enormous scale. However, the processes employed for the manufacture of commodity base chemicals is often environmentally unfriendly due to the production of waste streams, toxic gaseous side-products, high energy consumption and the like. An example of such a commodity base chemical is aniline. Aniline is mainly used for the manufacture of methylene diphenylisocyanate. Other uses include rubber-processing chemicals, herbicides, dyes and pigments, specialty fibres, explosives, epoxy curing agents and pharmaceuticals.
Most aniline is produced through nitration of benzene using a mixture of concentrated nitric acid and concentrated sulphuric acid followed by the hydrogenation of nitrobenzene, in the presence of a catalyst such as nickel, at about 600°C. Hydrogenation of nitrobenzene in the gas phase over solid catalysts is also known. Sunoco's aniline process (Halcon process), however, involves the conversion of phenol with ammonia, the phenol being manufactured from cumene, along with the formation of diphenylamine as a side-product.

Currently, many waste streams or rest streams which still comprise useful starting materials for valuable base chemicals including commodities are produced in the food industry and the bio-fuel industry. Many of such waste streams or rest streams include proteinaceous materials, e.g. proteins and/or amino acids. Such waste streams or rest streams could be converted into valuable base chemicals.

JP A 2135093 to Takasago Perfumery Co. Ltd., discloses the production of anthranilic acid, wherein a culture medium comprising tryptophan is inoculated with a strain Achromobacter where after the culture medium is shaken at 28°C for 48 h followed by work-up.

EP 549.421 A1 to Pernod-Ricard discloses the fermentation of apple or grape juice using Saccharomyces cerevisiae mutants which are auxothrophic for tryptophan to produce anthranilic acid. US 5,422,256 to BASF Aktiengesellschaft the fermentation of a nutrient solution comprising tryptophan with a strain of Bacillus subtilis which are auxothrophic for tryptophan to produce anthranilic acid. Takemura et al. [Takemura Y., Nakamura A., Taguchi H. (1985), "Catalytic decarboxylation of benzoic acid", Ind. Eng. Chem. Prod. Res. Dev. 24: 213-215] discloses the thermal decomposition of anthranilic acid at 250°C in the presence of a cerium cation exchanged zeolite catalyst which yielded about 90% aniline.

McCollough et al. [McCollough W., Piligan J.T., Daniel I. (1957), "Enzymatic decarboxylation of aminobenzoates", J. Am. Chem. Soc. 79: 628-630] discloses enzymatic decarboxylation benzoates by using cell free extracts from Escherichia coli cultures. When incubated with the substrate under aerobic conditions, about 50% aniline was obtained.

In Zhurnal Organicheskoi Chimii, vol 25, No. 2, 1989, pages 431 to 433, a process for the manufacturing of kynurenine by irradiating tryptophan with a 200W thungstun lamp is disclosed.

It is an object of the present invention to provide an economically viable process for conversion of tryptophan into kynurenine, the process having an acceptable conversion rate of the tryptophan, and an acceptable selectivity to kynurenine.

It is a further object of the present invention to provide an economically viable process for manufacturing aniline, which process can use bio-mass as raw material.

According to a first aspect of the present invention, a process for converting tryptophan into kynurenine is provided. The conversion of tryptophan into kynurenine is carried out by irradiating a substance comprising tryptophan with electromagnetic radiation comprising waves with wavelength in the range of 10 nanometer to 750 nanometer, the oxidation reaction being a photo-catalytic oxidation reaction, the photo-catalytic catalyst being based on transition metal oxides or transition metal sulphides.

The unit "nanometer" is hereinafter also referred to as nm.
Electromagnetic radiation (also referred to as EMR) with a wavelength in the range of 10 nm to 750 nm encompasses the UV and visible light spectrum.

EMR with a wavelength in the range of 750 nm to 380 nm is better known as the visible light spectrum, in which EMR with a wavelength in the range of 750 nm to 620 nm refers to red light, EMR with a wavelength in the range of 620 nm to 590 nm refers to orange light, EMR with a wavelength in the range of 590 nm to 570 nm refers to yellow light, EMR with a wavelength in the range of 570 nm to 495 nm refers to green light, EMR with a wavelength in the range of 495 nm to 450 nm refers to blue light and EMR with a wavelength in the range of 450 nm to 380 nm refers to violet light.
EMR with a wavelength in the range of 10 nm to 400 nm is better known as "UV light" or Ultraviolet radiation. The range comprises various sub-ranges, each being given a specific name. Ultraviolet A or long wave UV or black light refers to EMR with waves having a wavelength in the range of 400 nm to 320 nm. Near UV refers to EMR with waves having a wavelength in the range of 400 nm to 300 nm. Ultraviolet B or medium wave UV refers to EMR with waves having a wavelength in the range of 320 nm to 280 nm. Middle UV refers to EMR with waves having a wavelength in the range of 300 nm to 200 nm. Ultraviolet C or short wave UV or germicidal UV refers to EMR with waves having a wavelength in the range of 280 nm to 100 nm. Far UV refers to EMR with waves having a wavelength in the range of 200 nm to 122 nm. Vacuum UV refers to EMR with waves having a wavelength in the range of 200 nm to 10 nm. Extreme UV refers to EMR with waves having a wavelength in the range of 121 nm to 10 nm.

Illumination may be provided by a 150 W medium-pressure mercury-vapor lamp with 39% of total radiated energy in the UV spectrum between 250 - 380 nm.

This conversion of tryptophan into kynurenine is an oxidation reaction, which may be a photo-catalytic oxidation reaction using electromagnetic radiation, which radiation preferably has a wavelength in the range of 10 nm to 400 nm.

Preferably TiO₂ is used as catalyst in the photo-catalytic reaction.
Other suitable photo-catalytic catalysts are based on transition metal oxides or transition metal sulphides and include e.g. Fe₂O₃, V₂O₅, Bi₂AlVO₇, CdS/ZnS, Caln₂O₄, tantalum oxynitride, tungsten oxide, β-Ga₂O₃, Fe₂O₃/FeVO₄, CaZrTi₂O₇, and BiVO₄.

The TiO₂ photo-catalyst may be provided in various phases such as in any of rutile and anatase. The TiO₂ may be impregnated with other transition metals (such as V, Cr, Mn, Fe, Co, Ni, Cu, Zr, Nb, Ru, Rh, Ag, Cd, Ge, Ga, Zn, etc ...), and may be provided on different supports such as silica, alumina, silica/alumina, microporous supports, or mesoporous supports.

Possibly, though not necessarily, the photochemical or photo-catalytic oxidation reaction is performed in presence of air or oxygen.
The oxygen may be provided as oxygen dissolved naturally from the air into the solution under ambient conditions. Preferably air or oxygen is provided by bubbling air or oxygen into the reaction mixture.

According to some embodiments of the present invention, electromagnetic radiation having waves with wavelength in the Ultraviolet A range may be used, in particular when used in a photo-chemical reaction, optionally, even preferably with the presence of oxygen or air.

The photo-catalytic oxidation reaction may be performed in presence of an electric current flowing between at least two electrodes. Preferably, one of these electrodes, the anode, may comprise the photo-catalyst (e.g. TiO₂) where the two electrodes have a potential difference in the range of 0.5 V to 10 V.

The substance comprising tryptophan may be provided using a substance comprising proteinaceous material as raw material.
A waste or waste stream, a primary agricultural stream or the mixture thereof comprises 5 to 95 wt.% of proteinaceous material, based on the total weight of the waste, the primary agricultural stream or the mixture thereof may be used to provide as substance comprising proteinaceous material for providing the substance comprising tryptophan. More preferably, the waste, the primary agricultural stream or the mixture thereof comprises 10 to 90 wt.% of proteinaceous material and most preferably 15 to 75 wt.% Preferably, the waste, the primary agricultural stream or the mixture thereof is produced in the food industry, the bio-fuel industry, the biomass industry, the animal feed industry or the power generation industry, most preferably in the food industry or in the bio-fuel industry.
The proteinaceous material may comprise of proteins derived from animals, such as casein, fish meal, slaughterhouse residues, egg protein or protein derived from manure, specifically chicken manure, or may comprise of vegetable proteins, such as gluten or soy, such as soybean meal, rape press cake, sunflower seed presscake, leaf proteins, the pods and stems of sunflower, canola and soy, and/or amino acid residues, such as amino acid residues from industrial processing of beet, potato, corn, wheat and soy.

A substance comprising tryptophan may be obtained by hydrolysis of the substance comprising proteinaceous material.
The hydrolysis may preferably be performed with an enzyme or a combination of enzymes, wherein the enzymes are preferably selected from the group consisting of proteases and peptidases. The enzymes may be used in various degrees of purity and/or may be present within living, dead or denatured microorganisms, which microorganisms may be genetically modified.

According to another embodiment of the present invention, the hydrolysis is preferably performed with an acid or a base, most preferably a basic hydrolysis. Tryptophan shows great stability under conditions of basic hydrolysis. For example an aqueous solution of tryptophan (0.2 g/l) does not decompose even after 24 hours under reflux with 6N KOH or 6N NaOH at pH >13.
Optionally, the hydrolysis is preferably performed with a combination of an enzyme and an acid or a base.

The proteinaceous material may comprise a source for tryptophan and/or may comprise tryptophan.

As an alternative, the substance comprising tryptophan may be a product obtained from a fermentation process.

According to a second aspect of the present invention, a process for providing or manufacturing aniline is provided. This process comprises the steps of
1. providing a substance comprising tryptophan;
2. converting said tryptophan into kynurenine according to the first aspect of the present invention; and
3. converting said kynurenine into aniline.

Converting the kynurenine into aniline may be converting the kynurenine via anthranilic acid to aniline.

According to the present invention, converting the tryptophan into kynurenine is done by irradiating said substance comprising tryptophan which electromagnetic radiation comprising waves with wavelength in the range of 10 nm to 750 nm.

The conversion of kynurenine into aniline may be done by first subjecting the kynurenine to an enzymatic treatment, e.g. using kynureninase, providing a mixture comprising anthranilic acid and alanine. The anthranilic acid can be converted to aniline.

The conversion of kynurenine to aniline may be carried out by two steps. The first step is an enzymatic conversion of kynurenine to anthranilic acid and alanine. The enzyme used may be kynureninase, which is naturally produced in the liver of mammals. Other enzymes suitable to perform the enzymatic conversion of kynurenine to anthranilic acid and alanine may be used. The enzymes may be used in various degrees of purity and/or may be present within living, dead or denatured microorganisms, which microorganisms may be genetically modified.

As an example, an aqueous solution of kynurenine with a concentration of 0.2 g/l may be totally converted (100% conversion) to anthranilic acid and alanine within 8 minutes at room temperature. The substrate ratio was 1 kynurenine : 0.4 enzyme : 3 H₂O at pH 8.5. The enzyme used was kynureninase.

The anthranilic acid can be decarboxylated into aniline.
This decarboxylation step may be performed in the presence of an enzyme. The enzyme may be an enzyme obtained from Escherichia coli.

This decarboxylation step may be performed in the presence of an enzyme, such as the enzyme obtained from Escherichia coli [W. McCollough, J. T. Piligan, I. Daniel. J. Am. Chem. Soc., 79 (1957) 628]. A 25 ml of aqueous solution of anthranilic acid with a concentration of 100 micromole was 50% converted into aniline by the effect of 8 mg of bacterial N, at pH = 6 after 3 hours in 35°C under anaerobic conditions. The solution contained also 0.4 micromole of pyridoxal phosphate and 1.0 microatom of iron (III).

As an alternative, the decarboxylation can be a thermal reaction either with or without the presence of a catalyst, e.g. homogeneous or heterogeneous catalysts. Suitable homogeneous catalysts are H₂SO₄ or HCl at elevated temperature (e.g. ca. 100°C). Suitable heterogeneous catalysts are cerium cation exchanged zeolites, e.g. CeHY. If the reaction is performed in the presence of a heterogeneous catalyst or a homogeneous catalyst, this reaction is preferably performed at a temperature of 80°C to 500°C, more preferably at a temperature of 100°C to 450°C.

As a further alternative, the decarboxylation can be an acid based reaction, wherein the anthranilic acid is reacted with a mineral acid, preferably H₂SO₄ or HCl, or with acetic acid. The reaction is preferably performed at elevated temperatures, e.g. in the range of 100°C to 250°C.

In case the decarboxylation is an acid based reaction or a thermal reaction, aniline or aniline-water mixtures may be used as a solvent in which the anthranilic acid is brought in solution.

As a further alternative, the decarboxylation can be a photo-catalytic reaction, optionally using TiO₂, optionally at room temperature, and optionally using a suitable light source (e.g. medium pressure mercury-vapour lamps, black light,... etc).

The independent and dependent claims set out particular and preferred features of the invention. Features from the dependent claims may be combined with features of the independent or other dependent claims as appropriate.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention.
Figure 1 shows results of a photochemical conversion of tryptophan to kynurenine according to the first aspect of the present invention, the conversion being shown as a function of irradiation time either in the absence of air pumping / presence of air pumping.
Figure 2 and figure 3 show results of a photo-catalytic conversion of tryptophan to kynurenine according to the first aspect of the present invention. In figure 2, the tryptophan conversion is plotted against the reaction time. In figure 3, the conversion increase with increasing the TiO₂ amount present in the reactor is shown.
Figure 4 shows results of an alternative photo-catalytic conversion of tryptophan to kynurenine according to the first aspect of the present invention. Figure 4, shows the conversion of tryptophan in function of the light intensity used.
Figure 5 and figure 6 show results of a decarboxylation reaction of anthranilic acid into aniline, as part of a process for providing aniline according to the second aspect of the present invention. Figures 5 and 6 show respectively the conversion of the anthranilic acid and the selectivity towards aniline with reaction time.
Figure 7 shows a black-light reactor suitable for use in the method according to the present invention.
Figure 8 shows an emission curve of a UV-lamp suitable for use in the method according to the present invention.

The present invention will be described with respect to particular embodiments.
It is to be noticed that the term "comprising", used in the claims, should not be interpreted as being restricted to the means listed thereafter; it does not exclude other elements or steps. It is thus to be interpreted as specifying the presence of the stated features, steps or components as referred to, but does not preclude the presence or addition of one or more other features, steps or components, or groups thereof. Thus, the scope of the expression "a device comprising means A and B" should not be limited to devices consisting only of components A and B. It means that with respect to the present invention, the only relevant components of the device are A and B.
Throughout this specification, reference to "one embodiment" or "an embodiment" are made. Such references indicate that a particular feature, described in relation to the embodiment is included in at least one embodiment of the present invention. Thus, appearances of the phrases "in one embodiment" or "in an embodiment" in various places throughout this specification are not necessarily all referring to the same embodiment, though they could. Furthermore, the particular features or characteristics may be combined in any suitable manner in one or more embodiments, as would be apparent to one of ordinary skill in the art.

The following terms are provided solely to aid in the understanding of the invention.

When reference is made to percentages of a component, mole percentage of the component is meant, unless otherwise indicated. The mole percentage is expressed relative to the total number of moles of the substrate of composition in which the component is present.

In a first step of the present invention, a substance comprising tryptophan is provided. This substance may be obtained from proteinaceous material comprising proteins derived from animals, such as casein, fish meal, slaughterhouse residues, egg protein or protein derived from manure, specifically chicken manure, or may comprise of vegetable proteins, such as gluten or soy, such as soybean meal, rape press cake, sunflower seed presscake, leaf proteins, the pods and stems of sunflower, canola and soy, and/or amino acid residues, such as amino acid residues from industrial processing of beet, potato, corn, wheat and soy.

In this first example, the conversion of tryptophan to kynurenine is done using a photochemical reaction. 850 ml of an aqueous solution containing tryptophan (tryptophan concentration of 0.2 g/l) was converted in a photoreactor (obtained from Pschele Co. Germany) in the presence of oxygen. The solution was stirred for 2 hours with a stirring rate of 700-900 rpm, and a 150 W medium pressure mercury-vapour lamp with a UV range from 250 nm to 380 nm was illuminated in the solution.

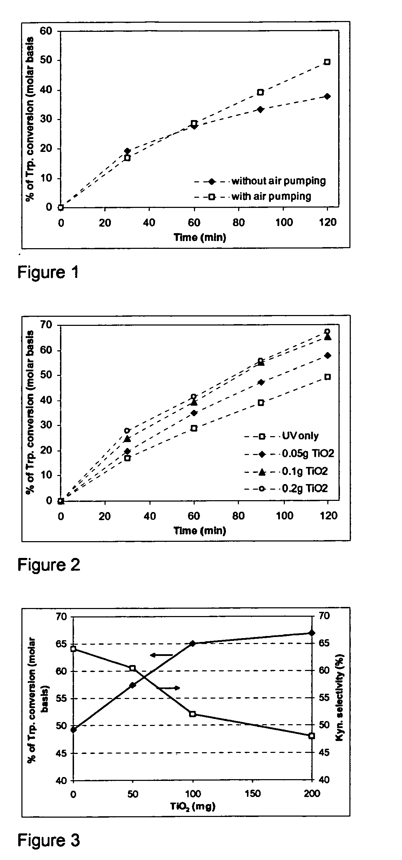

Samples of the resulting product were withdrawn on interval basis from the solution and analyzed directly by means of HPLC. Figure 1 shows the conversion of tryptophan as a function of irradiation time either in the absence/presence of air bubbling through the solution with a rate of 200-250 ml/min. The oxygen content of the air gas stream was consistent with normal air mixture (e.g. 20-22%).
Up to 37% of tryptophan is converted in absence of air, while in the presence of air almost 50% is converted. Selectivity towards kynurenine was 72% without air bubbles and 68% when air bubbles are provided.

In an alternative process for converting tryptophan into kynurenine according to the present invention, being a photo catalytic conversion, 850 ml of aqueous tryptophan solution (0.2 g/l) was placed in the same reactor that has been described above 50 mg of (P-25 of Degussa) TiO₂ was added to the solution containing tryptophan. Again, air was bubbled through the solution with a rate of 200-250 ml/min.
The solution was stirred for 20 minutes, where after the medium pressure mercury-vapour lamp was ignited and the solution was stirred with a rate of 700-900 rpm.

Samples were withdrawn on an interval basis and filtrated through micro-filters to remove TiO₂ and then analyzed by means of HPLC. In figure 2, the tryptophan conversion is plotted against the reaction time. Results obtained from the HPLC showed that almost 50% of tryptophan is converted within 2 hours, with a selectivity of 61 % towards kynurenine. In further tests, the reaction was repeated with different TiO₂ concentration (100 mg and 200 mg). Results, as shown in figure 3, are a conversion increase with increasing the TiO₂ amount, however the selectivity towards kynurenine decreases as a function of increasing the tryptophan conversion.

In an alternative photo catalytic reaction based process for converting tryptophan into kynurenine according to the present invention, a reactor (entitled black-light reactor) has been designed for this purpose.

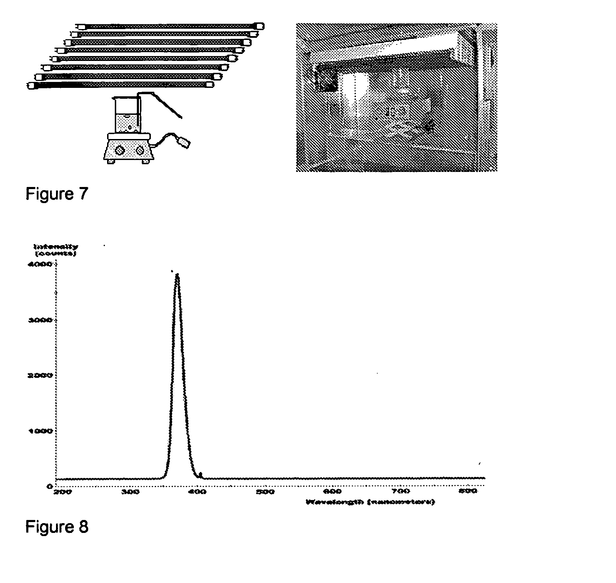

Figure 7 shows a picture of the 'black-light reactor'. The reactor is a closable aluminum box with dimensions of 60 cm height, 60 cm width, and 40 cm thickness. The box was equipped with a 12 V transformer for an electric exhausting fan. Eight lamps (F20 T8 BLB) emitting approximately 350-400 nm light (the emissions shown in figure 8), 18 W black-light lamps (60 cm x 2.5 cm) were used; each lamp was independently controlled by an ON/OFF button. The experiments were carried out in beakers (Schott Duran) with a length of 8 cm, an internal diameter of 6.2 cm, and a volume of 100 ml. Air was allowed to bubble during the experiments through a thin plastic tube with a rate of 200-250 ml/min.

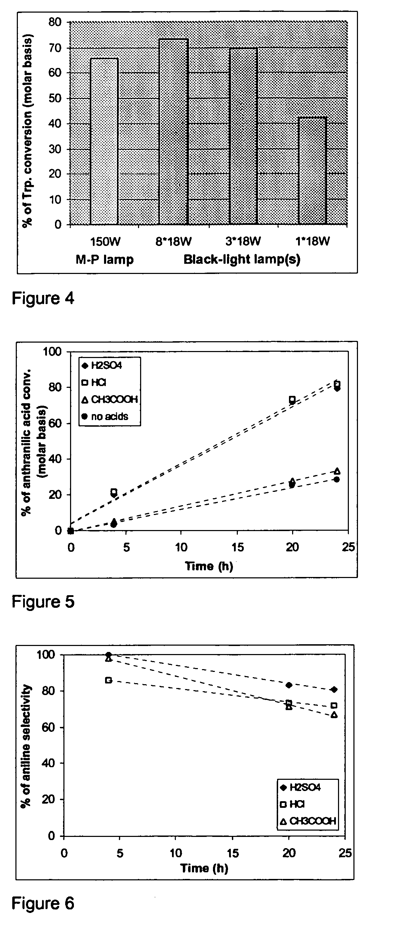

50 ml of an aqueous solution containing tryptophan with a concentration of 0.2 g/l was placed in the beaker, 50 mg of TiO₂ was added for photo-catalyzing the tryptophan conversion. The distance between the surface of tryptophan solution and the light sources was adjusted to be 5 cm. The tryptophan solution with TiO₂ was stirred first for 20 minutes, after which the black light lamps were switched on and the solution was stirred with a rate of 700-900 rpm.
Samples were withdrawn on an interval basis with a period of two hours. The samples were filtrated through micro-filters to remove TiO₂ and were analyzed by means of HPLC.
Results, as represented in figure 4, show a conversion of 42% of tryptophan with a 92% selectivity towards kynurenine when 1 black-light lamp was only used (1*18 W). When 3 lamps were used, 70% of tryptophan was converted with 90% selectivity towards kynurenine (3*18 W). When 8 lamps were used, 72% of tryptophan was converted with 90% selectivity towards kynurenine (8*18 W). When the medium pressure lamp was used, 65% of tryptophan was converted with 52% selectivity towards kynurenine (150 W).

In a subsequent step, the resulting products as obtained from one of the process steps above comprising kynurenine was subjected to a step for converting the kynurenine into anthranilic acid and alanine.
This conversion was 100% within 8 minutes. In a typical example: An aqueous solution of kynurenine with a concentration of 0.2 g/l was totally converted to anthranilic acid and alanine at room temperature when kynureninase enzyme was used. The substrates ratio was 1 kynurenine : 0.4 enzyme : 3 H₂O at pH 8.5.

In an subsequent step, the resulting products comprising anthranilic acid, after separation from the other components using conventional techniques, provided in an aqueous solution of 0.2 to 5 gram per liter, can be decarboxylated into aniline.

The resulting products comprising anthranilic acid was decarboxylated into aniline at 100°C under fluxing conditions.
An aqueous solution of anthranilic acid with a concentration of 0.2 g/l was prepared and 50 ml of the solution was placed in a round flask. The flask was heated to 100°C. Three experiments were done, wherein a mineral or organic acid was added (37 mmol/l of H₂SO₄, 65 mmol/l of HCl or acetic acid). One experiment was done without addition of an acid. Samples were withdrawn on interval basis, and analyzed by HPLC.
Results as reflected in figures 5 and 6, show that anthranilic acid was decarboxylated to aniline either in the presence of acids or not. 27% of anthranilic acid was converted after 24 hours without acids. However, in the presence of a mineral acid, such as in the experiments H₂SO₄ or HCl, almost 80% of conversion was obtained with 80% and 71% a selectivity towards aniline, respectively.

In an alternative process, the resulting products comprising anthranilic acid was decarboxylated using ultraviolet illumination in the presence of a photocatalyst, typically TiO₂.
In an example, an aqueous solution of anthranilic acid was prepared and it was photo-oxidized with TiO₂ at room temperature. The light source was either the medium pressure mercury-vapour lamp or the black-light lamps as described above. Air was bubbled through the solution with a rate of 200-250 ml/min.
The conversion reached 15% and then the formed aniline was photo-oxidized further to carbon dioxide and water.

## Claims

1. A process for converting tryptophan into kynurenine, wherein converting tryptophan into kynurenine is carried out by irradiating a substance comprising tryptophan with electromagnetic radiation comprising waves with wavelength in the range of 10 nanometer to 750 nanometer, said oxidation reaction is a photo-catalytic oxidation reaction, the photo-catalytic catalyst being based on transition metal oxides or transition metal sulphides.

2. A process according to claim 1, wherein the photo-catalytic catalyst includes Fe₂O₃, V₂O₅, Bi₂AlVO₇, CdS/ZnS, Caln₂O₄, tantalum oxynitride, tungsten oxide, β-Ga₂O₃, Fe₂O₃/FeVO₄, CaZrTi₂O₇ or BiVO₄.

3. A process according to claim 1, wherein said oxidation reaction is a photo-catalytic oxidation reaction using TiO₂ as photo-catalyst.

4. A process according to claim 3, wherein the TiO₂ photo-catalyst is provided as rutile and anatase.

5. A process according to claim 3 or 4, wherein the TiO₂ photo-catalyst is impregnated with other transition metals selected from the group consisting of V, Cr, Mn, Fe, Co, Ni, Cu, Zr, Nb, Ru, Rh, Ag, Cd, Ge, Ga and Zn.

6. A process according to any one of the claims 3 to 5, wherein the TiO₂ photo-catalyst is provided on a support selected from the group consisting of silica, alumina, silica/alumina, microporous supports and mesoporous supports.

7. A process according to any one of the claims 43 to 6, wherein said photo-catalytic oxidation reaction is performed in presence of electromagnetic radiation having waves with wavelength in the Ultraviolet A range.

8. A process according to any one of the claims 3 to 7, wherein said photo-catalytic oxidation reaction is performed in presence of an electric current flowing between at least two electrodes.

9. A process according to any one of the claims 1 to 8, wherein the tryptophan is provided from a substance comprising proteinaceous material.

10. A process according to any one of the claims 1 to 9, wherein the tryptophan is provided by means of hydrolysis of a proteinaceous material.

11. A process according to claim 10, wherein the tryptophan is provided by means of a basic hydrolysis of a proteinaceous material.

12. A process for providing or manufacturing aniline, the process comprises the steps of
1. providing a substance comprising tryptophan;
2. converting said tryptophan into kynurenine according to any one of the claims 1 to 11; and
3. converting said kynurenine into aniline.

13. A process according to claim 12, wherein the conversion of kynurenine into aniline comprises subjecting the kynurenine to an enzymatic treatment, for providing a mixture comprising anthranilic acid and alanine, and converting said anthranilic acid to aniline.

14. A process according to claim 12, wherein the conversion of kynurenine into aniline comprises subjecting the kynurenine to an enzymatic treatment using kynureninase for providing a mixture comprising anthranilic acid and alanine, and converting said anthranilic acid to aniline.

15. A process according to claim 13 or 14, wherein the anthranilic acid is decarboxylated into aniline.

16. A process according to claim 15, wherein decarboxylation is provided by a thermal reaction with the presence of a catalyst.

17. A process according to claim 15, wherein decarboxylation is provided by thermal reaction without the presence of a catalyst.

18. A process according to claim 15, wherein decarboxylation is provided by an acid based reaction.

19. A process according to claim 15, wherein decarboxylation is provided by an acid based reaction using H₂SO₄, HCl or acetic acid.

20. A process according to claim 18 or 19, wherein said anthranilic acid is brought in aniline or an aniline-water based solution.

21. A process according to claim 15, wherein decarboxylation is provided by a photo-catalytic reaction.

## Patentansprüche

1. Verfahren zum Umwandeln von Tryptophan in Kynurenin, wobei das Umwandeln von Tryptophan in Kynurenin durch Bestrahlen einer Tryptophan aufweisenden Substanz mit elektromagnetischen Strahlen erfolgt, die Wellen mit einer Wellenlänge im Bereich von 10 bis 750 nm aufweisen, wobei die Oxidationsreaktion eine photokatalytische Oxidationsreaktion ist und der Photokatalysator auf Übergangsmetalloxiden oder Übergangsmetallsulfiden basiert.

2. Verfahren nach Anspruch 1,
wobei der Photokatalysator Fe₂O₃, V₂O₅, Bi₂AlVO₇, CdS/ZnS, CaIn₂O₄, Tantaloxinitrid, Wolframoxid, β-Ga₂O₃, Fe₂O₃/FeVO₄, CaZrTi₂O₇ oder BiVO₄ aufweist.

3. Verfahren nach Anspruch 1,
wobei die Oxidationsreaktion eine photokatalytische Oxidationsreaktion ist, die TiO₂ als Photokatalysator verwendet.

4. Verfahren nach Anspruch 3,
wobei der TiO₂-Photokatalysator als Rutil und Anatas bereitgestellt wird.

5. Verfahren nach Anspruch 3 oder 4,
wobei der TiO₂-Pholokatalysator mit anderen Übergangsmetallen imprägniert wird, die aus der Gruppe ausgewählt sind, die aus V, Cr, Mn, Fe, Co, Ni, Cu, Zr, Nb, Ru, Rh, Ag, Cd, Ge, Ga und Zn besteht.

6. Verfahren nach einem der Ansprüche 3 bis 5,
wobei der TiO₂-Photokatalysator auf einem Träger bereitgestellt wird, der aus der Gruppe ausgewählt ist, die aus Siliciumdioxid, Aluminiumoxid, Siliciumdioxid/Aluminiumoxid, mikroporösen Trägern und mesoporösen Trägern besteht.

7. Verfahren nach einem der Ansprüche 3 bis 6,
wobei die photokatalytische Oxidationsreaktion in Gegenwart von elektromagnetischen Strahlen mit Wellen mit einer Wellenlänge im UV-A-Bereich erfolgt.

8. Verfahren nach einem der Ansprüche 3 bis 7,
wobei die photokatalytische Oxidationsreaktion in Gegenwart von elektrischem Strom erfolgt, der zwischen mindestens zwei Elektroden fließt.

9. Verfahren nach einem der Ansprüche 1 bis 8,
wobei das Tryptophan von einer Substanz bereitgestellt wird, die ein proteinhaltiges Material aufweist.

10. Verfahren nach einem der Ansprüche 1 bis 9,
wobei das Tryptophan durch Hydrolyse eines proteinhaltigen Materials bereitgestellt wird.

11. Verfahren nach Anspruch 10,
wobei das Tryptophan durch basische Hydrolyse eines proteinhaltigen Materials bereitgestellt wird.

12. Verfahren zum Bereitstellen oder Herstellen von Anilin,
wobei das Verfahren die folgenden Schritte aufweist:
1. Bereitstellen einer Tryptophan aufweisenden Substanz;
2. Umwandeln des Tryptophans in Kynurenin nach einem der Ansprüche 9 bis 11; und
3. Umwandeln des Kynurenins in Anilin.

13. Verfahren nach Anspruch 12,
wobei das Umwandeln von Kynurenin in Anilin das Unterziehen des Kynurenins einer Enzymbehandlung, um ein Anthranilsäure und Alanin aufweisendes Gemisch bereitzustellen, und das Umwandeln dieser Anthranilsäure in Anilin aufweist.

14. Verfahren nach Anspruch 12,
wobei das Umwandeln von Kynurenin in Anilin das Unterziehen des Kynurenins einer Enzymbehandlung unter Verwendung von Kynureninase, um ein Anthranilsäure und Alanin aufweisendes Gemisch bereitzustellen, und das Umwandeln dieser Anthranilsäure in Anilin aufweist.

15. Verfahren nach Anspruch 13 oder 14,
wobei die Anthranilsäure zu Anilin decarboxyliert wird.

16. Verfahren nach Anspruch 15,
wobei das Decarboxylieren durch thermische Reaktion in Gegenwart eines Katalysators erfolgt.

17. Verfahren nach Anspruch 15,
wobei das Decarboxylieren durch eine thermische Reaktion erfolgt, ohne dass ein Katalysator vorhanden ist.

18. Verfahren nach Anspruch 15,
wobei das Decarboxylieren durch eine auf Säure basierende Reaktion erfolgt.

19. Verfahren nach Anspruch 15,
wobei das Decarboxylieren durch eine auf Säure basierende Reaktion erfolgt, wobei H₂SO₄, HCl oder Essigsäure verwendet wird.

20. Verfahren nach Anspruch 18 oder 19,
wobei die Anthranilsäure in eine auf Anilin oder Anilin-Wasser basierende Lösung eingebracht wird.

21. Verfahren nach Anspruch 15,
wobei das Decarboxylieren durch eine photokatalytische Reaktion erfolgt.

## Revendications

1. Procédé pour la conversion de tryptophane en kynurénine, dans lequel la conversion du tryptophane en kynurénine est effectuée en irradiant une substance comprenant du tryptophane avec un rayonnement électromagnétique comprenant des ondes ayant une longueur d'onde dans l'intervalle de 10 nanomètres à 750 nanomètres, ladite réaction d'oxydation est une réaction d'oxydation photocatalytique, le catalyseur photocatalytique étant à base d'oxydes de métaux de transition ou de sulfures de métaux de transition.

2. Procédé suivant la revendication 1, dans lequel le catalyseur photocatalytique comprend Fe₂O₃, V₂O₅, Bi₂AlVO₇, CdS/ZnS, Caln₂O₄, l'oxynitrure de tantale, l'oxyde de tungstène, β-Ga₂O₃, Fe₂O₃/FeVO₄, CaZrTi₂O₇ ou BiVO₄.

3. Procédé suivant la revendication 1, dans lequel ladite réaction d'oxydation est une réaction d'oxydation photocatalytique utilisant du TiO₂ comme photocatalyseur.

4. Procédé suivant la revendication 3, dans lequel le photocatalyseur TiO₂ est fourni sous forme de rutile et d'anatase.

5. Procédé suivant la revendication 3 ou 4, dans lequel le photocatalyseur TiO₂ est imprégné d'autres métaux de transition choisis dans le groupe consistant en V, Cr, Mn, Fe, Co, Ni, Cu, Zr, Nb, Ru, Rh, Ag, Cd, Ge, Ga et Zn.

6. Procédé suivant l'une quelconque des revendications 3 à 5, dans lequel le photocatalyseur TiO₂ est fourni sur un support choisi dans le groupe consistant en la silice, l'alumine, la silice/alumine, des supports micro-poreux et des supports mésoporeux.

7. Procédé suivant l'une quelconque des revendications 3 à 6, dans lequel ladite réaction d'oxydation photo-catalytique est effectuée en présence d'un rayonnement électromagnétique ayant des ondes d'une longueur d'onde dans la plage du rayonnement ultraviolet A.

8. Procédé suivant l'une quelconque des revendications 3 à 7, dans lequel ladite réaction d'oxydation photo-catalytique est effectuée en présence d'un courant électrique passant entre au moins deux électrodes.

9. Procédé suivant l'une quelconque des revendications 1 à 8, dans lequel le tryptophane est fourni à partir d'une substance comprenant une matière protéique.

10. Procédé suivant l'une quelconque des revendications 1 à 9, dans lequel le tryptophane est fourni par hydrolyse d'une matière protéique.

11. Procédé suivant la revendication 10, dans lequel le tryptophane est fourni par une hydrolyse basique d'une matière protéique.

12. Procédé pour fournir ou produire de l'aniline, le procédé comprenant les étapes consistant
1. à fournir une susbtance comprenant du tryptophane ;
2. à convertir ledit tryptophane en kynurénine suivant l'une quelconque des revendications 1 à 11 ; et
3. à convertir ladite kynurénine en aniline.

13. Procédé suivant la revendication 12, dans lequel la conversion de la kynurénine en aniline comprend l'étape consistant à soumettre la kynurénine à un traitement enzymatique pour fournir un mélange comprenant de l'acide anthranilique et de l'alanine, et à convertir ledit acide anthranilique en aniline.

14. Procédé suivant la revendication 12, dans lequel la conversion de la kynurénine en aniline comprend l'étape consistant à soumettre la kynurénine à un traitement enzymatique en utilisant de la kynuréninase pour fournir un mélange comprenant de l'acide anthranilique et de l'alanine, et à convertir ledit acide anthranilique en aniline.

15. Procédé suivant la revendication 13 ou 14, dans lequel l'acide anthranilique est dicarboxylé en aniline.

16. Procédé suivant la revendication 15, dans lequel la décarboxylation est réalisée par une réaction thermique en présence d'un catalyseur.

17. Procédé suivant la revendication 15, dans lequel la décarboxylation est réalisée par une réaction thermique sans la présence d'un catalyseur.

18. Procédé suivant la revendication 15, dans lequel la décarboxylation est effectuée par une réaction basée sur un acide.

19. Procédé suivant la revendication 15, dans lequel la décarboxylation est réalisée par une réaction basée sur un acide, utilisant H₂SO₄, HCl ou l'acide acétique.

20. Procédé suivant la revendication 18 ou 19, dans lequel ledit acide anthranilique est transformé en aniline ou en une solution aqueuse à base d'aniline.

21. Procédé suivant la revendication 15, dans lequel la décarboxylation est effectuée par une réaction photo-catalytique.
